# EUROPEAN PATENT APPLICATION

(11) **EP 4 427 733 A1**
(43) Date of publication of application: **11.09.2024**
(21) Application number: 23743446.9
(22) Date of filing: 18.01.2023
(51) Int. Cl.: A61K 8/9789, A61Q 19/00, A61K 36/53, A61P 17/00, A61P 17/02, A61P 17/14

(54) **NOVEL COMPOSITION COMPRISING NEPETA CARTARIA-DERIVED EXOSOMES AS ACTIVE INGREDIENT**

(30) Priority: 24.01.2022 KR 20220010217; 17.01.2023 KR 20230006863
(71) Applicant: Exocobio Inc., Cheongju-si, Chungcheongbuk-do 28161 (KR)
(72) Inventor: CHO, Byong Seung, Anyang-si Gyeonggi-do 14102 (KR)
(74) Representative: Isarpatent
(86) International application number: PCT/KR2023/000834
(87) International publication number: WO 2023/140599

(57) **Abstract**

The present invention provides a composition for skin elasticity improvement, skin wrinkle reduction, skin regeneration, anti-inflammation, wound healing, or wound healing acceleration, containing exosomes derived from *Nepeta cataria* as an active ingredient. The composition according to the present invention is less likely to contain impurities such as residual solvents and the like compared to conventional hot-water extracts of *Nepeta cataria,* solvent extracts of *Nepeta cataria,* filtrates of such extracts, and has excellent effects on skin elasticity improvement, skin wrinkle reduction, skin regeneration, anti-inflammation, wound healing, or wound healing acceleration.

## Description

### TECHNICAL FIELD

The present invention relates to a novel composition comprising exosomes derived from *Nepeta cartaria* as an active ingredient, and more specifically, to a composition for skin elasticity improvement, skin wrinkle reduction, skin regeneration, anti-inflammation, wound healing or wound healing acceleration comprising exosomes derived from *Nepeta cataria* as an active ingredient.

Moreover, the present invention relates to a cosmetic composition for skin elasticity improvement, skin wrinkle reduction or skin regeneration comprising said composition, and to a pharmaceutical composition for anti-inflammation, wound healing or wound healing acceleration comprising said composition.

### BACKGROUND ART

It is known that skin aging leads to a decrease in skin elasticity and an increase in skin wrinkles and that the decrease in skin elasticity and the formation of skin wrinkles occur due to decreased synthesis of collagen and stimulated expression of the collagenase matrix metalloproteinase (MMP).

In addition, it is known that in skin cells, COX-2, an enzyme that produces inflammatory cytokines increases due to aging progression or ultraviolet (UV) rays, resulting in increased synthesis of prostaglandin E2 and increased production of inflammation inducers. Due to inflammatory reactions, the biosynthesis of MMP increases, causing collagen degradation and resulting in the decrease in skin elasticity and the formation of skin wrinkles. In particular, when sunlight and ultraviolet rays are irradiated directly onto skin, a lot of free radicals are generated, and these free radicals could damage the antioxidant defense system of skin, thus increasing wrinkles, making skin loose and accelerating skin aging. Substances known to be effective in reducing skin wrinkles include adenosine and retinoic acid. However, adenosine has little efficacy in clinical practice, and retinoic acid cannot be used for pregnant women and has side effects such as erythema.

Inflammation is a defense response of the body against physical or chemical injury, infection with bacteria, fungi or viruses, or pathological conditions caused by various allergens and the like. Inflammatory response appears as part of innate immune response. Various substances and physiological and chemical phenomena are involved in inflammatory response, and recent studies have shown that various inflammatory cytokines play an important role in inflammatory response. Major cytokines involved in inflammatory response include IL-1β, TNF-α, IL-6, IL-8, IL-12, IFN-β, and the like. The increased expression and secretion of these cytokines and the activation thereof are associated with a series of complex physiological responses, including secretion of inflammatory mediators, immune cell infiltration, cell migration, and tissue destruction, as well as symptoms such as erythema, edema, fever and pain.

In general, inflammatory response does not become a significant problem and the affected area returns to its normal state, if the infectious agent is removed from the body and the damaged tissue is regenerated. However, if the infectious agent is not removed from the body or the inflammatory response is excessive or persistent due to internal substances of the body, acute or chronic inflammatory disease occurs. Non-steroidal anti-inflammatory drugs, steroidal anti-inflammatory drugs, antagonists of neuropeptides, COX inhibitors, antihistamines, and immunosuppressive drugs such as cyclosporine A are used for alleviation or treatment of inflammatory response or inflammatory diseases caused thereby, but have problems that they cause adverse effects such as skin atrophy, vasodilation, depigmentation, hypersensitivity reactions, tolerance, neutropenia and the like. In addition, there is a limit that the aforesaid drugs merely help to control symptoms related to inflammation to a certain level rather than the underlying treatment therefor.

Recently, there have been reports that cell secretomes contain various bioactive molecules that regulate cellular behaviors. In particular, cell secretomes contain 'exosome' that has intercellular signaling functions, and thus studies on the components and functions thereof have been actively conducted.

Cells shed various membraneous vesicles to their extracellular environment, and these released vesicles are usually called extracellular vesicles (EVs). The EV is also called cell membrane-derived vesicle, ectosome, shedding vesicle, microparticle, exosome, etc., and is also used discriminately from exosome in some cases.

Exosome is a vesicle of tens to hundreds of nanometers in size, which comprises a phospholipid bilayer membrane having the same structure as that of the cell membrane. This exosome contains proteins, nucleic acids (mRNA, miRNA, etc.) and the like which are called exosome cargo. It is known that exosome cargo includes a wide range of signaling factors, and these signaling factors are specific for cell types and regulated differently depending on secretory cells' environment. It is known that exosome is an intercellular signaling mediator secreted by cells, and various cellular signals transmitted through it regulate cellular behaviors, including the activation, growth, migration, differentiation, dedifferentiation, apoptosis, and necrosis of target cells. Exosome contains specific genetic materials and bioactive factors depending on the nature and state of cells from which the exosome was derived. Exosome derived from proliferating stem cells regulates cell behaviors such as cell migration, proliferation and differentiation, and recapitulates the characteristics of stem cells involved in tissue regeneration (Nature Review Immunology 2002 (2) 569-579).

That is, exosomes called "avatars" of cells contain bioactive factors such as growth factors, similar to cells, and serve as carriers that transmit bioactive factors between cells, that is, serve to mediate cell-to-cell communication. Exosomes are known to be released not only from animal cells such as stem cells, immune cells, fibroblasts and cancer cells, but also from cells of various organisms such as plants, bacteria, fungi, and algae. For example, exosomes may be isolated from culture media of plant cells or plant stem cells, as well as culture media of cancer cells, immune cells, mesenchymal stem cells, and the like.

However, studies on the isolation, purification, and characterization of exosomes derived from plant cells or plant stem cells remain insufficient, and most of these studies are merely used for marketing purposes where extracellular vesicles mixed in the filtrate of plant juice are simply called exosomes. Therefore, more detailed characterization and functional studies of exosomes derived from plant cells are required.

Catnip, with the scientific name of *Nepeta cartaria,* is a perennial, herbaceous plant belonging to the genus Nepeta in the family Lamiaceae and the order Lamiales. *Nepeta cartaria* is a favorite of cats and commonly referred to as catnip. *Nepeta cataria* is a type of herb that, when dried, has a minty flavor and is also known as catmint. *Nepeta cataria* is native to Asia and Europe, and is distributed in Korea, Japan, China, Central Asia, Africa, Europe and North America. It is known for its pain-relieving and antipyretic properties. However, current technology remains at the level of using extracts, obtained by hot water extraction or solvent extraction of leaves, stems or whole grass of *Nepeta cataria,* as a food or cosmetic ingredient. However, solvent extracts have the problem of being toxic to the human body due to residual extraction solvents.

The present inventor has found that exosomes derived from *Nepeta cataria* are effective in skin elasticity improvement, skin wrinkle reduction, skin regeneration, anti-inflammation, wound healing or wound healing acceleration, and has developed a cosmetic composition for skin elasticity improvement, skin wrinkle reduction or skin regeneration and a pharmaceutical composition for anti-inflammation, wound healing or wound healing acceleration, containing exosomes derived from *Nepeta cataria* as an active ingredient.

Meanwhile, it is to be understood that the matters described as the background art are intended merely to aid in the understanding of the background of the present invention and are not admitted as prior art against the present invention.

### SUMMARY OF INVENTION

An object of the present invention is to provide a composition for skin elasticity improvement, skin wrinkle reduction, skin regeneration, anti-inflammation, wound healing or wound healing acceleration, containing exosomes derived from *Nepeta cartaria* as an active ingredient.

Another object of the present invention is to provide a cosmetic composition for skin elasticity improvement, skin wrinkle reduction or skin regeneration comprising said composition, and a pharmaceutical composition for anti-inflammation, wound healing or wound healing acceleration comprising said composition.

However, the objects of the present invention as described above are illustrative and the scope of the present invention is not limited thereby. In addition, other objects and advantages of the present invention will be more apparent from the following description, the appended claims and the accompanying drawings.

### DETAILED DESCRIPTION OF INVENTION

The present invention provides a composition for skin elasticity improvement, skin wrinkle reduction, skin regeneration, anti-inflammation, wound healing or wound healing acceleration, containing exosomes derived from *Nepeta cartaria* as an active ingredient.

As used herein, the term "catnip" refers to a perennial, herbaceous plant belonging to the genus Nepeta in the family Lamiaceae and the order Lamiales, and its scientific name is *Nepeta cartaria. Nepeta cataria* is a type of herb that, when dried, has a minty flavor and is also known as catmint. *Nepeta cataria* is native to Asia and Europe, and is distributed in Korea, Japan, China, Central Asia, Africa, Europe and North America.

As used herein, the term "exosomes" refers to nano-sized vesicles secreted or released from plant cells into extracellular spaces and having a membrane structure, and is also referred to as exosome-like vesicles or exosome-like particles.

As used herein, the term "skin elasticity" refers to a feature in which skin deformed by an external force easily returns to its original shape when the external force is removed. The term "skin wrinkles" refers to fine lines caused by skin aging. Skin wrinkles may be caused by genetic factors, reduction in collagen and elastin present in the skin dermis, external environmental factors, or the like. Accordingly, the term "skin wrinkle reduction or improvement" as used herein refers to suppressing or inhibiting the formation of wrinkles on the skin, or reducing already formed wrinkles.

As used herein, the term "anti-inflammation" means prevention, suppression, alleviation, amelioration or treatment of inflammation. As an example, not limiting the present invention, examples of inflammatory diseases include dermatitis, atopic dermatitis, eczema, inflammation caused by bacterial, viral or fungal infections, burns, inflammation caused by burns, wounds, inflammation caused by wounds, and the like.

As used herein, the term "wound" means a condition in which a part or all of the body is injured, and is intended to encompass pathological conditions in which a tissue constituting an inside or an external surface of the body, for example, skin, muscle, nerve tissue, bone, soft tissue, internal organ, or blood vessel tissue, is damaged or destroyed. As an example, not limiting the present invention, examples of the wound include abrasion, laceration, stab wound, incised wound, avulsion, bedsore, tissue destruction caused by irradiation, penetrated wound, gunshot wound, burn, frostbite, surgical wound, sutures after plastic surgery, wound caused by chemical substance and so on, and may include any damage to any part of an individual.

As used herein, the term "iontophoresis" refers to a method of flowing a microcurrent through a skin to which an active ingredient has been applied, generating a potential difference thereby and changing the electrical environment of the skin, and thus allowing an ionized active ingredient to penetrate the skin by electrical repulsion. Examples of iontophoresis that is used in one embodiment of the present invention include: a method of introducing a microcurrent into a skin by allowing the microcurrent to flow from an external power source into an electrode patch on the skin, the microcurrent being generated by the external power source; a method of introducing a microcurrent into a skin, the microcurrent being generated by a battery provided in an electrode patch on the skin; and a method of introducing a microcurrent into a skin through a patch on the skin provided with a reverse electrodialysis device, the microcurrent being generated by the concentration difference between high concentration electrolyte solution and low concentration electrolyte solution in the reverse electrodialysis device. However, the present invention is not limited thereto, and various types of iontophoresis may, of course, be used.

As used herein, the term "exosomes derived from *Nepeta cartaria"* is meant to include all exosomes isolated from, for example, a culture medium of *Nepeta cataria* plant cells, a culture medium of *Nepeta cataria* callus, a culture medium of *Nepeta cataria* plant stem cells, a *Nepeta cataria* juice, or a biological solution of *Nepeta cataria* equivalent thereto, or derived (e.g., secreted and/or released) from plant cells or plant stem cells of *Nepeta cataria.*

In the composition according to one embodiment of the present invention, the exosomes may isolated and purified from a *Nepeta cataria* juice.

The composition containing exosomes derived from *Nepeta cartaria* as an active ingredient according to one embodiment of the present invention may exhibit at least one effect of skin elasticity improvement, skin wrinkle reduction, skin regeneration, anti-inflammation, wound healing or wound healing acceleration.

The composition containing exosomes derived from *Nepeta cartaria* as an active ingredient according to the present invention may be a cosmetic composition or a pharmaceutical composition.

The present invention provides a cosmetic composition for skin elasticity improvement, skin wrinkle reduction or skin regeneration, containing exosomes derived from *Nepeta cartaria* as an active ingredient. For example, the cosmetic composition may be a shampoo, a soap, a rinse, a surfactant-containing cleanser, a cream, a lotion, an ointment, a tonic, a treatment, a conditioner, a suspension, an emulsion, a paste, a gel, an oil, a wax, a spray, an aerosol, a mist, or a powder, and is preferably a lotion or a cream.

The present invention also provides a pharmaceutical composition for anti-inflammation, wound healing or wound healing acceleration, containing exosomes derived from *Nepeta cartaria* as an active ingredient.

As an example not limiting the present invention, the pharmaceutical composition according to one embodiment of the present invention may be administered or treated by injection, microneedling, iontophoresis, application, or a combination thereof. For example, the pharmaceutical composition may be an injectable formulation, an infusion formulation, a spray formulation, a liquid formulation, or a patch formulation.

In one embodiment of the present invention, when the composition is used as a pharmaceutical composition, it may include pharmaceutically acceptable carriers, excipients or diluents. The carriers, excipients and dilutes include, but are not limited to, lactose, dextrose, trehalose, sucrose, sorbitol, mannitol, xylitol, erythritol, maltitol, starch, acacia gum, alginate, gelatin, calcium phosphate, calcium carbonate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. In addition, the effective amount of the pharmaceutical composition according to one embodiment of the present invention means the amount required for administration in order to achieve the effects of anti-inflammation, wound healing or wound healing acceleration.

The content of the pharmaceutical composition according to one embodiment in a formulation may be suitably selected depending on the kind, amount, form and the like of additional components as described above. For example, the pharmaceutical composition of the present invention may be contained in an amount of about 0.1 to 99 wt%, preferably about 10 to 90 wt%, based on the total weight of an injectable formulation. Furthermore, the suitable dose of the pharmaceutical composition according to one embodiment of the present invention may be adjusted depending on the severity of disease, the type of formulation, formulating method, patient's age, sex, body weight, health condition, diet, excretion rate, the period of administration, and the regime of administration. For example, when the pharmaceutical composition according to one embodiment of the present invention is administered to an adult, it may be administered once to several times at a dose of 0.001 mg/kg to 100 mg/kg per day.

Meanwhile, when the composition according to one embodiment of the present invention is prepared as a cosmetic composition, it may suitably contain components which are generally used in cosmetic products, for example, moisturizers, antioxidants, oily components, UV absorbers, emulsifiers, surfactants, thickeners, alcohols, powder components, colorants, aqueous components, water, and various skin nutrients, etc., as needed, within the range that does not impair the effect of the present invention.

Furthermore, the cosmetic composition according to one embodiment of the present invention may include, in addition to the exosomes derived from *Nepeta cartaria,* an agent for improving skin condition and/or a moisturizer, which have been used in the prior art, within the range that does not impair the effects (e.g., skin condition improvement, skin elasticity improvement, skin wrinkle reduction, skin regeneration, etc.).

The cosmetic composition according to one embodiment of the present invention may be used in various forms, for example, a patch, a mask pack, a mask sheet, a cream, a tonic, an ointment, a suspension, an emulsion, a paste, a lotion, a gel, an oil, a pack, a spray, an aerosol, a mist, a foundation, a powder, and an oilpaper.

The cosmetic composition according to one embodiment of the present invention may be used for the purpose of skin elasticity improvement, wrinkle improvement, skin regeneration and the like, and the cosmetic composition may be prepared as any formulation which is generally prepared in the art. For example, it may be formulated as patch, mask pack, mask sheet, skin softener, nutrition, astringent lotion, nourishing cream, massage cream, eye cream, cleansing cream, essence, eye essence, cleansing lotion, cleansing foam, cleansing water, sunscreen, lipstick, soap, shampoo, surfactant-containing cleanser, bath preparation, body lotion, body cream, body oil, body essence, body cleanser, hairdye, hair tonic, etc., but is not limited thereto.

The cosmetic composition according to one embodiment of the present invention may contain components which are commonly used in cosmetic products. For example, the cosmetic composition may contain conventional adjuvants and carriers, such as antioxidants, stabilizers, solubilizers, vitamins, pigments, and fragrances. In addition, other components in each formulation for the cosmetic composition may be suitably selected without difficulty by those skilled in the art depending on the type or intended use of the cosmetic composition.

Another embodiment of the present invention provides a cosmetic method for regulating mammalian skin conditions, except for treatment purposes, using the cosmetic composition. In the cosmetic method of the present invention, the expression "regulating skin conditions" means improving skin conditions and/or prophylactically regulating skin conditions, and the expression "improving skin conditions" means a visually and/or tactilely perceivable positive change in the appearance and feeling of skin. For example, the expression "improving skin conditions" may include skin wrinkle reduction, skin elasticity improvement or skin regeneration.

The cosmetic method according to one embodiment of the present invention includes: (a) applying the cosmetic composition directly to a mammalian skin; or (b) contacting or attaching a patch, a mask pack or a mask sheet, which has the cosmetic composition applied thereto or soaked therein, to the mammalian skin; or sequentially performing (a) and (b). In step (a), the cosmetic composition may be a lotion or a cream.

Alternatively, the cosmetic method according to one embodiment of the present invention may further comprise (c) removing the patch, the mask pack or the mask sheet from the mammalian skin after step (b), and applying the cosmetic composition to the mammalian skin. In step (c), the cosmetic composition may be a lotion or a cream.

As an example, not limiting the present invention, the cosmetic composition may be applied to a skin by microneedling, iontophoresis, direct application, or a combination thereof. For example, the cosmetic composition may be a spray formulation, a liquid formulation, or a patch formulation.

In the cosmetic method according to one embodiment of the present invention, the mammal may be a cat, a human, a dog, a rodent, a horse, a cattle, a monkey, or a pig.

In addition, the present invention provides a method for treating inflammation, healing wound or accelerating wound healing, the method comprising administering a therapeutically effective amount of the pharmaceutical composition to a mammal, or applying the pharmaceutical composition to a skin, an inflammatory area, or a wounded area.

As an example, not limiting the present invention, the pharmaceutical composition may be administered by injection, microneedling, iontophoresis, or a combination thereof. For example, the pharmaceutical composition may be an injectable formulation, an infusion formulation, a spray formulation, a liquid formulation, or a patch formulation.

The mammal may be a cat, a human, a dog, a rodent, a horse, a cattle, a monkey, or a pig.

### ADVANTAGEOUS EFFECTS

The composition according to the present invention is less likely to contain impurities such as residual solvents and the like compared to conventional hot-water extracts of *Nepeta cataria,* solvent extracts of *Nepeta cataria,* filtrates of such extracts, and has excellent effects on skin elasticity improvement, skin wrinkle reduction, skin regeneration, anti-inflammation, wound healing or wound healing acceleration.

It should be understood that the scope of the present invention is not limited to the aforementioned effects.

### BRIEF DESCRIPTION OF DRAWINGS

FIG. 1 is a graph showing the particle size distribution and particle number obtained by performing nanoparticle tracking analysis (NTA) of the exosomes derived from *Nepeta cataria* of the present invention.
FIG. 2 depicts fluorescence microscopic images of cells showing that fluorescence-stained exosomes derived from *Nepeta cataria* were delivered into human dermal fibroblasts (green: exosomes delivered into cells; and blue: cell nucleus).
FIG. 3 is a graph showing that the relative amount of collagen increased in a concentration-dependent manner after human dermal fibroblasts were treated with exosomes derived from *Nepeta cataria.*
FIG. 4 is a graph showing that the migration of human dermal fibroblasts increased after scratch-wounds were treated with exosomes derived from *Nepeta cataria.*
FIG. 5 depicts fluorescence microscopic images of cells showing that fluorescence-stained exosomes derived from *Nepeta cataria* were delivered into RAW 264.7 cells (green: exosomes delivered into cells; and blue: cell nucleus).
FIG. 6 is a graph showing that IL-6 induced by LPS decreased when RAW 264.7 cells were treated with exosomes derived from *Nepeta cataria.*

### EXAMPLES

Hereinafter, the present invention will be described in more detail with reference to the following examples. However, the following examples are only to illustrate the present invention and are not intended to limit or restrict the scope of the present invention. Those that can be easily inferred by those skilled in the art from the detailed description and examples of the present invention are interpreted as falling within the scope of the present invention. References referred to in the present invention are incorporated herein by reference.

Throughout the present specification, it is to be understood that, when any part is referred to as "comprising" any component, it does not exclude other components, but may further include other components, unless otherwise specified.

### Example 1: Preparation of Nepeta cataria Juice

The leaves and stems of *Nepeta cartaria* were first washed under running water to remove pesticides. Next, the leaves and stems were cut into pieces of no more than 5 cm, and juiced with a low-speed screw at a stirring speed of 40 rpm to obtain a liquid juice, which was then filtered through a 400-mesh net to remove large suspended materials. The filtered juice was centrifuged at 10,000 × g for 10 minutes at 4°C to remove proteins and cell walls. The supernatant obtained by the above procedure was filtered through a filter membrane with 0.22 µm pores to obtain a filtrate, which was stored at -80°C until purification.

### Example 2: Purification of Nepeta cataria-Derived Exosomes

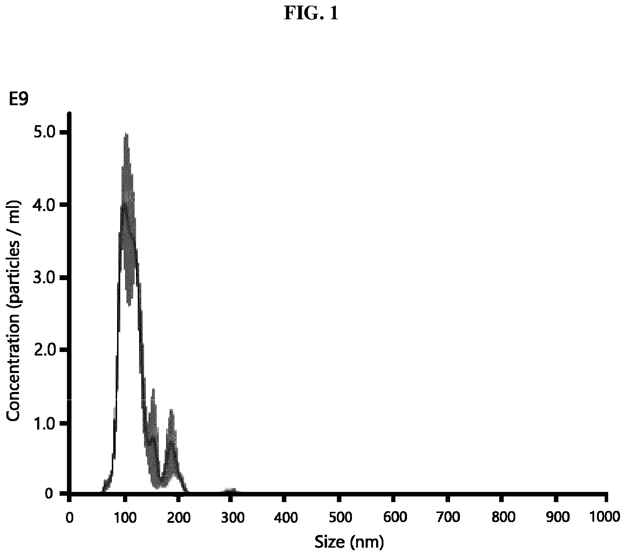

The *Nepeta cataria* juice prepared as described in Example 1 was subjected to stepwise centrifugation and ultracentrifugation to remove a number of impurities and to isolate *Nepeta cataria*-derived exosomes. The thawed *Nepeta cataria* juice was centrifuged at 2,000×g for 10 min and filtered through a filter membrane with 0.22 µm pores to obtain a filtrate. The filtrate underwent first centrifugation at 10,000×g and second centrifugation at 20,000×g, and then a supernatant was recovered. The supernatant was ultracentrifuged at 100,000×g for 70 min at 4°C to obtain a pellet. The obtained pellet was suspended in phosphate-buffered saline (PBS) to finally obtain exosomes derived from *Nepeta cataria.* The size and concentration of *Nepeta cataria*-derived exosomes isolated by the above method were analyzed by nanoparticle tracking analysis (NTA) using NS300 (purchased from Malvern Panalytical) (FIG. 1).

### Example 3: Evaluation of Delivery Ability of Nepeta cataria-Derived Exosomes into Dermal Fibroblasts

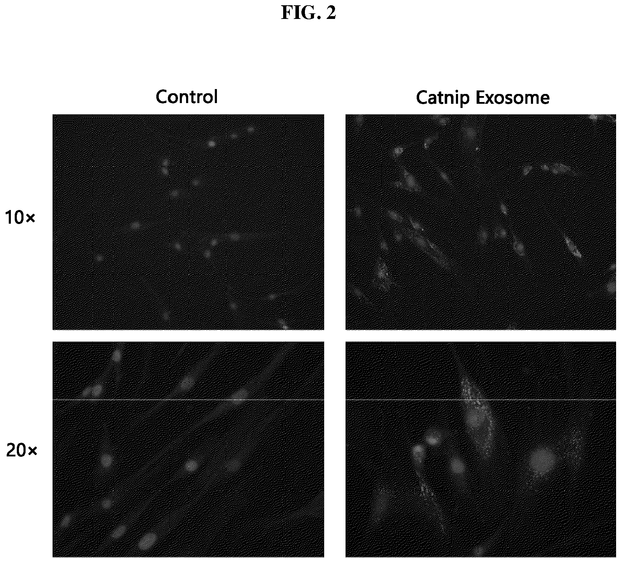

In order to examine whether *Nepeta cataria*-derived exosomes would be delivered into human dermal fibroblasts (purchased from ATCC), the following analysis was performed. To fluorescence-stain the membrane of *Nepeta cataria*-derived exosomes prepared in Example 2, the exosomes were allowed to react with PKH67 fluorescence dye (purchased from Sigma-Aldrich). After the reaction, the reaction solution was fractionated with a MiniTrap-25 column (purchased from Cytiva) to remove free PHK67 that was not stained in the exosome membrane. A negative control was prepared by allowing PKH67 fluorescence dye to react with a buffered solution and fractionating the reaction product with a MiniTrap-25 column. The exosomes stained with PKH67 were incubated with pre-cultured human dermal fibroblasts, and then whether the exosomes would be delivered into the cells over time was observed using a fluorescence microscope. Hoechst fluorescence dye (purchased from Thermo Fisher) was used to stain the cell nucleus, and CellMask Orange fluorescence dye (purchased from Thermo Fisher) was used to stain the cytoplasm. As a result of examining whether the exosomes would be delivered into the cells, it was confirmed that the fluorescence-stained exosomes were delivered into the cells and green fluorescence accumulated in the cells over time (FIG. 2).

### Example 4: Evaluation of Effect of Stimulating Collagen Production

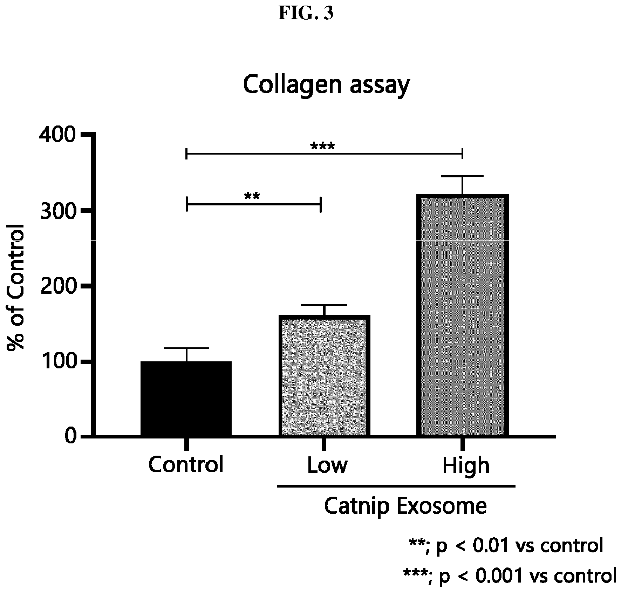

Human dermal fibroblasts (purchased from ATCC) dispersed in a DMEM medium containing fetal bovine serum were dispensed into a multiwell plate, and then cultured for 24 hours. The human dermal fibroblasts were further cultured in serum-free medium for 48 hours. Thereafter, *Nepeta cataria*-derived exosomes prepared in Example 2 were diluted in serum-free medium, and then the human dermal fibroblasts were treated with each of the dilutions and cultured. To evaluate the collagen production effect using human dermal fibroblasts, experimental groups were classified as follows:
(1) Negative control group (Control): an experimental group treated with a serum-free medium alone;
(2) Group treated with a low concentration of *Nepeta cataria*-derived exosomes (indicated as "Low" in FIG. 3): an experiment group treated with *Nepeta cataria*-derived exosomes (prepared in Example 2) diluted in serum-free medium (treatment concentration: 1.0×10¹⁰ particles/mL); and
(3) Group treated with a high concentration of *Nepeta cataria*-derived exosomes (indicated as "High" in FIG. 3): an experiment group treated with *Nepeta cataria*-derived exosomes (prepared in Example 2) diluted in serum-free medium (treatment concentration: 4.0×10¹⁰ particles/mL).

According to each experimental group described above, the human dermal fibroblasts were treated and cultured for 24 hours. The culture media were collected and centrifuged, and then the centrifuged media were prepared. The amount of collagen, which was synthesized from the human dermal fibroblasts and accumulated in the culture media, was measured using an EIA kit (purchased from Takara) for procollagen type I C-peptide (PIP). The measured amount of collagen was normalized by dividing it by the total number of cells measured using an MTT assay kit (purchased from Sigma-Aldrich) to determine the relative amount of collagen.

As a result, it was confirmed that *Nepeta cataria*-derived exosomes of the present invention remarkably increased collagen synthesis in the human dermal fibroblasts in a concentration-dependent manner, compared to the negative control (FIG. 3).

From the above experimental results, it can be seen that *Nepeta cataria*-derived exosomes of the present invention have an excellent effect of increasing collagen synthesis, that is, skin elasticity improvement, wrinkle reduction and/or skin regeneration.

As can be seen from the above results, *Nepeta cataria*-derived exosomes of the present invention has a useful functional activity as a functional cosmetic for skin elasticity improvement, wrinkle reduction and/or skin regeneration, that is, an activity of increasing collagen synthesis. Thus, *Nepeta cataria*-derived exosomes of the present invention are useful as an active ingredient of a cosmetic composition for skin elasticity improvement, wrinkle reduction and/or skin regeneration.

### Example 5: Evaluation of Skin Regeneration Effect Using Dermal Fibroblasts

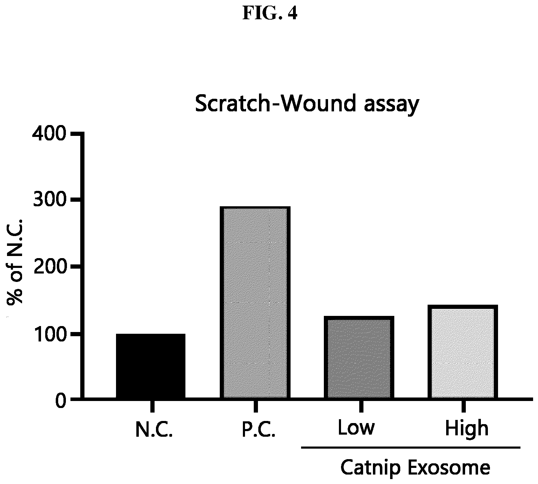

To evaluate whether *Nepeta cataria*-derived exosomes prepared as described in Example 2 promotes wound healing in human dermal fibroblasts (purchased from ATCC), scratch-wound assay was performed. Human dermal fibroblasts dispersed in a DMEM containing fetal bovine serum were dispensed into a culture plate for wound induction (ImageLock Plate; purchased from EssenBio) at a density of 5,000 cells/well and cultured for 24 hours at 37°C under 5% CO₂. After the cells reached a confluency of 90% or more, scratches were made using a WoundMaker (purchased from EssenBio). To evaluate the skin regeneration effect using human dermal fibroblasts, experimental groups were classified as follows:
(1) Negative control group (N.C.): an experimental group treated with a serum-free medium alone;
(2) Positive control group (P.C.): an experimental group treated with a medium containing 10% fetal bovine serum;
(3) Group treated with a low concentration of *Nepeta cataria*-derived exosomes (indicated as "Low" in FIG. 4): an experiment group treated with *Nepeta cataria*-derived exosomes (prepared in Example 2) diluted in serum-free medium (treatment concentration: 2.0×10⁹ particles/mL); and
(4) Group treated with a high concentration of *Nepeta cataria*-derived exosomes (indicated as "High" in FIG. 4): an experiment group treated with *Nepeta cataria*-derived exosomes (prepared in Example 2) diluted in serum-free medium (treatment concentration: 1.2×10¹⁰ particles/mL).

Thereafter, each of the experimental groups was subjected to Scratch-Wound, and the human dermal fibroblasts were cultured at 37°C under 5% CO₂ for 12 hours. The wound healing efficacy in each experimental group was measured using Incucyte (purchased from Sartorius).

As a result of measuring the wound healing efficacy, it was confirmed that *Nepeta cataria*-derived exosomes of the present invention increased the migration of the human dermal fibroblasts, compared to the negative control (FIG. 4).

As can be seen from the above experimental results, *Nepeta cataria*-derived exosomes of the present invention have an excellent effect of promoting the migration of human dermal fibroblasts, that is, an excellent wound healing effect or skin regeneration effect.

Therefore, *Nepeta cataria*-derived exosomes of the present invention are useful as an active ingredient of a cosmetic composition for skin elasticity improvement, wrinkle reduction and/or skin regeneration, and as an active ingredient of a pharmaceutical composition for wound healing or wound healing acceleration.

### Example 6: Evaluation of Delivery Ability of Nepeta cataria-Derived Exosomes into Macrophages

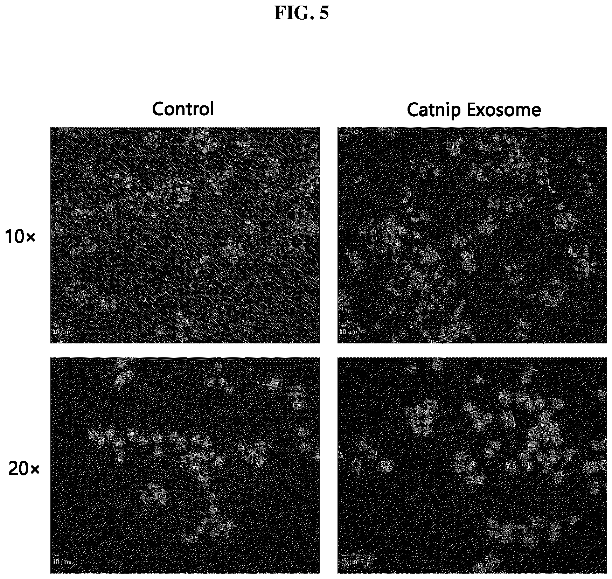

In order to examine whether *Nepeta cataria*-derived exosomes would be delivered into mouse macrophages (RAW 264.7; purchased from ATCC), the following analysis was performed. To fluorescence-stain the membrane of *Nepeta cataria*-derived exosomes prepared in Example 2, the exosomes were allowed to react with PKH67 fluorescence dye (purchased from Sigma-Aldrich). After the reaction, the reaction solution was fractionated with a MiniTrap-25 column (purchased from Cytiva) to remove free PHK67 that was not stained in the exosome membrane. A negative control was prepared by allowing PKH67 fluorescence dye to react with a buffered solution and fractionating the reaction product with a MiniTrap-25 column. The exosomes stained with PKH67 were incubated with pre-cultured mouse macrophages, and then whether the exosomes would be delivered into the cells over time was observed using a fluorescence microscope. Hoechst fluorescence dye (purchased from Thermo Fisher) was used to stain the cell nucleus, and CellMask Orange fluorescence dye (purchased from Thermo Fisher) was used to stain the cytoplasm. As a result of examining whether the exosomes would be delivered into the cells, it was confirmed that the fluorescence-stained exosomes were delivered into the cells and green fluorescence accumulated in the cells over time (FIG. 5).

### Example 7: Evaluation of Anti-Inflammatory Effect of Nepeta cataria-Derived Exosomes

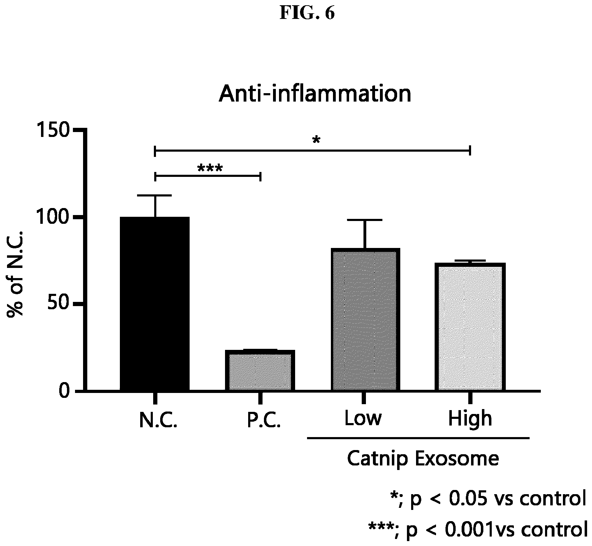

In order to examine whether *Nepeta cataria*-derived exosomes prepared as described in Example 2 exhibit an anti-inflammatory effect, the effect of *Nepeta cataria*-derived exosomes on the amount of IL-6 production in RAW 264.7 cells, which are mouse macrophages, was evaluated. RAW 264.7 cells suspended in a DMEM medium containing 10% FBS were dispensed into each well of a multiwell plate to reach a confluency of 80 to 90%. The next day, the RAW 264.7 cells were treated with *Nepeta cataria*-derived exosomes diluted in an LPS-containing fresh medium [DMEM containing 1% FBS and 200 nM LPS (lipopolysaccharide)], and then the RAW 264.7 cells were cultured for 24 hours. Experimental groups for evaluating the anti-inflammatory effect were classified as follows.
(1) Negative control group (N.C.): an experimental group in which RAW 264.7 cells were treated with an LPS-containing medium alone;
(2) Positive control group (P.C.): an experimental group in which RAW 264.7 cells were treated with an LPS-containing medium mixed with dexamethasone (final concentration: 200 µM);
(3) Group treated with a low concentration of *Nepeta cataria*-derived exosomes (indicated as "Low" in FIG. 6): an experiment group in which RAW 264.7 cells were treated with *Nepeta cataria*-derived exosomes (prepared in Example 2) diluted in an LPS-containing medium (treatment concentration: 6.0×10⁹ particles/mL); and
(4) Group treated with a high concentration of *Nepeta cataria*-derived exosomes (indicated as "High" in FIG. 6): an experiment group in which RAW 264.7 cells were treated with *Nepeta cataria*-derived exosomes (prepared in Example 2) diluted in an LPS-containing medium (treatment concentration: 2.0×10¹⁰ particles/mL).

After completing the culturing of the RAW 264.7 cells in each experimental group, the culture supernatant was collected, and the production amount of the inflammatory cytokine IL-6 present in the culture supernatant was measured using an IL-6 ELISA kit. The amount of IL-6 (inflammatory cytokine) produced in the group treated with LPS alone, and the amount of IL-6 produced in each of the experimental groups treated with the LPS-containing media mixed with dexamethasone and *Nepeta cataria*-derived exosomes (low concentration and high concentration exosomes), respectively, were measured using an ELISA kit (purchased from R&D Systems) according to the manufacturer's manual (FIG. 6).

As a result, it was confirmed that in the experimental groups in which the RAW 264.7 cells were treated with LPS plus *Nepeta cataria*-derived exosomes (low concentration and high concentration exosomes) prepared in Example 2, the production of IL-6 was inhibited compared to that in the negative control group (FIG. 6).

As can be seen from the above experimental results, *Nepeta cataria*-derived exosomes of the present invention have an excellent anti-inflammatory effect. Thus, *Nepeta cataria-*derived exosomes of the present invention are useful as an active ingredient of a pharmaceutical composition for anti-inflammation, wound healing and/or wound healing acceleration.

Although the present invention has been described with reference to the embodiments, the scope of the present invention is not limited to these embodiments. Any person skilled in the art will appreciate that various modifications and changes are possible without departing from the spirit and scope of the present invention and these modifications and changes also fall within the scope of the present invention.

## Claims

1. A cosmetic composition for skin elasticity improvement, skin wrinkle reduction, skin regeneration, comprising exosomes derived from *Nepeta cartaria* as an active ingredient.

2. The cosmetic composition of claim 1, wherein the composition is a shampoo, a soap, a rinse, a surfactant-containing cleanser, a cream, a lotion, an ointment, a tonic, a treatment, a conditioner, a suspension, an emulsion, a paste, a gel, an oil, a wax, a spray, an aerosol, a mist, or a powder.

3. The cosmetic composition of claim 1 or 2, wherein the exosomes are isolated from a culture medium of *Nepeta cataria* plant cells, a culture medium of *Nepeta cataria* callus, a culture medium of *Nepeta cataria* plant stem cells, a *Nepeta cataria* juice, or a biological solution of *Nepeta cataria* equivalent thereto, or derived from plant cells or plant stem cells of *Nepeta cataria.*

4. The cosmetic composition of claim 3, wherein the exosomes are isolated and purified from the *Nepeta cataria* juice.

5. A pharmaceutical composition for anti-inflammation, wound healing or wound healing acceleration, comprising exosomes derived from *Nepeta cartaria* as an active ingredient.

6. The pharmaceutical composition of claim 5, wherein the composition is administered or treated by injection, microneedling, iontophoresis, application, or a combination thereof.

7. The pharmaceutical composition of claim 5, wherein the composition is an injectable formulation, an infusion formulation, a spray formulation, a liquid formulation, or a patch formulation.

8. The pharmaceutical composition of any one of claims 5 to 7, wherein the exosomes are isolated from a culture medium of *Nepeta cataria* plant cells, a culture medium of *Nepeta cataria* callus, a culture medium of *Nepeta cataria* plant stem cells, a *Nepeta cataria* juice, or a biological solution of *Nepeta cataria* equivalent thereto, or derived from plant cells or plant stem cells of *Nepeta cataria.*

9. The pharmaceutical composition of claim 8, wherein the exosomes are isolated and purified from the *Nepeta cataria* juice.

10. A cosmetic method for skin elasticity improvement, skin wrinkle reduction or skin regeneration to a mammalian skin, except for treatment purposes, using a cosmetic composition comprising exosomes derived from *Nepeta cartaria* as an active ingredient.

11. The cosmetic method of claim 10, wherein the method comprising:
(a) applying the cosmetic composition directly to the mammalian skin; or (b) contacting or attaching a patch, a mask pack or a mask sheet, which has the cosmetic composition applied thereto or soaked therein, to the mammalian skin; or sequentially performing (a) and (b).

12. The cosmetic method of claim 11, wherein the cosmetic composition is a lotion or a cream in step (a).

13. The cosmetic method of claim 11 or 12, further comprising step (c) removing the patch, the mask pack or the mask sheet from the mammalian skin after step (b), and applying the cosmetic composition to the mammalian skin.

14. The cosmetic method of claim 13, wherein the cosmetic composition is a lotion or a cream in step (c).

15. The cosmetic method of any one of claims 10 to 12, wherein the mammal is a cat, a human, a dog, a rodent, a horse, a cattle, a monkey, or a pig.

16. A method for treating inflammation, healing wound or accelerating wound healing, the method comprising administering a therapeutically effective amount of a pharmaceutical composition comprising exosomes derived from *Nepeta cartaria* as an active ingredient to a mammal except for a human, or applying a pharmaceutical composition comprising exosomes derived from *Nepeta cartaria* as an active ingredient to a skin, an inflammatory area, or a wounded area of a mammal except for a human.

17. The method of claim 16, wherein the pharmaceutical composition is administered by injection, microneedling, iontophoresis, or a combination thereof.

18. The method of claim 16, wherein the pharmaceutical composition is an injectable formulation, an infusion formulation, a spray formulation, a liquid formulation, or a patch formulation.

19. The method of any one of claims 16 to 18, wherein the mammal is a cat, a dog, a rodent, a horse, a cattle, a monkey, or a pig.
